# EUROPEAN PATENT APPLICATION

(11) **EP 3 913 003 A1**
(43) Date of publication of application: **24.11.2021**
(21) Application number: 20740833.7
(22) Date of filing: 15.01.2020
(51) Int. Cl.: C08B 37/16, C08G 81/00, A61P 7/04, A61K 47/40, A61K 45/00

(54) **CUBIC CYCLODEXTRIN FRAMEWORK-RGD COMPOSITION AND PREPARATION METHOD THEREFOR**

(30) Priority: 17.01.2019 CN 201910045633
(71) Applicant: Shanghai Institute of Materia Medica, Chinese Academy of Sciences, Pudong, Shanghai 201203 (CN)
(72) Inventor: ZHANG, Jiwen, Shanghai 201203 (CN); HE, Yaping, Shanghai 201203 (CN); SU, Yong, Shanghai 201203 (CN); XU, Jian, Shanghai 201203 (CN); WU, Li, Shanghai 201203 (CN); SUN, Xian, Shanghai 201203 (CN)
(74) Representative: Neumann, Ditmar
(86) International application number: PCT/CN2020/072261
(87) International publication number: WO 2020/147754

(57) **Abstract**

A cubic cyclodextrin framework-RGD composition (RGD-COF) and a preparation method therefor. Specifically, the cyclodextrin framework-RGD composition contains a cyclodextrin framework (COF) having a cubic structure and RGD, and can escape from phagocytosis and clearance of macrophages, enhance migration and adhesiveness to damaged blood vessels, and efficiently target and aggregate activated platelets at damaged blood vessel sites, having application prospects on targeted diagnosis and treatment of diseases associated with uncontrolled hemorrhage, atherosclerosis and cerebral apoplexy. By utilizing the advantage that a cyclodextrin-metal organic framework (CD-MOF) has controllable dimensions, a cubic cyclodextrin framework-RGD composition for nanoscale intravenous injection or microscale topically external use is provided.

## Description

### Technical Field

The invention relates to the field of biological materials, and more particularly to a cubic cyclodextrin framework-RGD composition and preparation method therefor.

### Background

The vascular related diseases, such as myocardial infarction and stroke, have a high morbidity and mortality all over the world. Non-targeted agents, such as urokinase and other thrombolytic drugs, have some problems, such as bleeding side effects and wide systemic exposure. Due to the complex hemodynamics of blood circulation system and the rapid clearance of normal carriers, targeted delivery of therapeutic drugs or contrast agents to vascular injury sites still faces great challenges. The physical and chemical properties of nano-carriers, such as shape, will affect the fate and biological function of nano-carriers *in vivo.* Recent studies have shown that non-spherical drug carriers can evade the body clearance mechanism, prolong the circulation time and increase the adhesion to the blood vessel surface. The study of controllable, targeted and non-spherical morphological carriers has brought new hope for targeted diagnosis and efficient treatment of vascular related diseases.

The mortality caused by uncontrolled blood loss is high in hospitals and battlefields. Normal physiological hemostasis process is far from enough in the case of serious injury, such as car accident or war trauma. Effective and rapid hemostasis and reducing bleeding time are important measures to reduce patient mortality. At present, the hemostatic materials commonly used in clinic, such as hemostatic gauze, hemostatic fiber and hemostatic bandage, have deficiencies. These hemostatic materials can only be used for external bleeding, need a long time to achieve hemostasis, and is easy to adhere to the wound and is difficult to change dressing, and is ineffective for infection and suppuration of the wound. Internal bleeding is mostly caused by organ rupture, and it is almost impossible to stop bleeding "at the first time" after injury because hemostatic drugs for external application can not be used. Recombinant human coagulation factor VII (rFVII) is the representative of systemic hemostatic drugs, but rFVII is expensive, easy to be inactivated and difficult to preserve, which greatly limits its clinical application. Platelet products have some defects, such as immunogenicity, difficult storage and easy inactivation, which also limit their application in emergency treatment. Therefore, intravenous hemostatic materials for the treatment of internal bleeding have great clinical demand. In summary, it is urgent to develop hemostatic materials with faster hemostatic speed and better effect, and which can be used for internal bleeding.

The specific binding of RGD sequence with GPIIb/IIIa receptor on the activated platelet surface is the final pathway of hemostatic clot formation. The RGD sequence can only bind to the activated platelet at the bleeding site, and resting platelets in normal blood circulation have no GPIIb/IIIa receptors on their surface, so there is no effect on circulating platelets. In recent years, it has become a new research direction for researchers to construct artificial platelets using polymer materials loaded with RGD polypeptide. Lavik's team used PLGA-PLL as a polymer carrier, and attached GRGDS pentapeptide onto PLGA-PLL-PEG to construct a synthetic platelet. The artificial platelet was administered by intravenous, which could activate platelets, promote platelet aggregation and further trigger coagulation mechanism, and the bleeding time was reduced by 45% in rat femoral artery injury model. However, the artificial platelet has some shortcomings, e.g. synthesis method is complex. Anirban Sen Gupta's team modified nanoliposomes with annular RGD to construct an artificial platelets, which reduced the bleeding time by about 50% in the mouse tail transection model after injected through tail vein. Mitragotri's team simulated the morphology and rheology of platelets by modifying PAH-BSA nanoparticles with GRGDS, and the synthesized artificial platelets could reduce the bleeding time in mouse tail transection model by 45%. Zhang Jianxiang's team injected positively charged nanoparticles synthesized by cholic acid and polyethyleneimine into the tail vein, and reduced the bleeding time by about 40% in the rat femoral artery injury model. Tan Yingxia et al. invented an artificial platelet PLGA-PEG-RGD by loading PLGA-PEG nanoparticles with RGD. PLGA-PEG-RGD is regular spherical, but its particle size is uneven. Although PLGA-PEG-RGD can be used as nano-hemostatic material for vein, the hemostatic effect in rat liver injury model is limited, and the hemostatic time can only be reduced by about 30%.

The hemostatic efficiency of artificial platelet depends largely on the surface biological functions and physical and mechanical properties of nanoparticles, such as size and shape. The morphology of nano-carriers not only affects their dynamic migration to the blood vessel wall, but also affects their adhesion and aggregation interaction with activated platelets. Compared with spherical particles, carriers with anisotropic shapes (such as ellipsoids and rods) have higher migration ability to blood vessel walls and stronger adhesion in physiologically flow environments. In addition, the geometry of the carrier will also affect its circulation and clearance *in vivo.* Therefore, regulating the shape of carrier brings new hope for overcoming physiological barrier and improving hemostatic efficiency.

However, most previous studies focused on spherical carriers, and no one has ever tried to use a cubic carrier for targeted therapy of hemorrhage.

### Summary of Invention

The purpose of the invention is to provide a cyclodextrin framework-RGD composition (RGD-COF) and a preparation method therefor and uses thereof.

In the first aspect of the present invention, it provides a cyclodextrin framework-RGD composition in which the mass ratio of the cyclodextrin framework to RGD is 1: 0.001-1: 1, and preferably 1: 0.005-1: 0.5; and
the cyclodextrin framework-RGD composition has a particle size of 10 nm-50 µm, preferably 50 nm-50 µm, more preferably 100-500 nm or 1-5 µm.

In another preferred embodiment, the composition is a cuboidal or cubic cyclodextrin framework-RGD composition.

In another preferred embodiment, the mass ratio of cyclodextrin framework to RGD in the cuboidal cyclodextrin framework-RGD composition is 1: 0.001-1: 1, preferably 1: 0.005-1: 0.5, and more preferably 1: 0.04-1: 0.5.

In another preferred embodiment, the mass ratio of cyclodextrin framework to RGD in the cuboidal cyclodextrin framework-RGD composition is 1: 0.005-1: 0.1, and preferably 1: 0.05.

In another preferred embodiment, the mass ratio of cyclodextrin framework to RGD in the cuboidal cyclodextrin framework-RGD composition is 1: 0.05.

In another preferred embodiment, the mass ratio of cyclodextrin framework to RGD in the cuboidal cyclodextrin framework-RGD composition is 1: 0.049, 1: 0.08, 1: 0.005, 1: 0.015, 1: 0.05, 1: 0.016, 1: 0.065, or 1: 0.046.

In another preferred embodiment, the cuboidal cyclodextrin framework-RGD composition has a particle size of 50 nm to 50 µm.

In another preferred embodiment, the cuboidal cyclodextrin framework-RGD composition has a particle size of 100-500 nm.

In another preferred embodiment, the cuboidal cyclodextrin framework-RGD composition has a particle size of 100-300 nm, and preferably 150-200 nm.

In another preferred embodiment, the cuboidal cyclodextrin framework-RGD composition has a particle size of 1-50 µm, preferably 30-50 µm, more preferably 10-30 µm.

In another preferred embodiment, the cuboidal cyclodextrin framework-RGD composition has a particle size of 1-5 µm.

In another preferred embodiment, the cuboidal cyclodextrin framework-RGD composition has a particle size of 200-500 nm, 100-300 nm, 200-400 nm, 200-500 nm, 1-10 µm, 1-5 µm, 30-50 µm, or 10-30 µm.

In another preferred embodiment, the RGD comprises a linear RGD or a circular RGD.

In another preferred embodiment, the RGD is a linear RGD.

In another preferred embodiment, the RGD is selected from the group consisting of RGD, GRGD, RGDS, RGDV, RGDF, GRGDV, GRGDF, GRGDS, RGDDSP, RGDDAP, other polypeptides containing the RGD sequence, and combinations thereof.

In another preferred embodiment, the linear RGD is selected from the group consisting of linear RGD, linear GRGD, linear RGDS, linear GRGDS, and combinations thereof.

In another preferred embodiment, the annular RGD is selected from the group consisting of annular RGD, annular GRGD, annular RGDS, annular RGDV, annular RGDF, annular GRGDV, annular GRGDF, annular GRGDS, annular RGDDSP, annular RGDDAP, other polypeptides containing annular RGD sequences, and combinations thereof.

In another preferred embodiment, the cyclodextrin is selected from group consisting of α-cyclodextrin (alpha-cyclodextrin), β-cyclodextrin (beta-cyclodextrin), γ-cyclodextrin (gamma-cyclodextrin), hydroxypropyl-β-cyclodextrin, sulfobutyl-β-cyclodextrin, methyl-β-cyclodextrin, carboxymethyl-β-cyclodextrin, and combinations thereof.

In another preferred embodiment, the cyclodextrin is selected from group consisting of α-cyclodextrins, β-cyclodextrins, γ-cyclodextrins, and more preferably γ-cyclodextrins.

In another preferred embodiment, the cyclodextrin is γ-cyclodextrin.

In another preferred embodiment, the cyclodextrin framework-RGD composition reduces the clot formation time by more than 30%, 40%, 50%, 60%, 70%, 80% or 90%.

In another preferred embodiment, the cyclodextrin framework-RGD composition reduces the bleeding time by more than 50%, 60%, 70%, 80%, 85%, 90% or 95%.

In another preferred embodiment, the cyclodextrin framework-RGD composition reduces the blood loss by more than 50%, 60%, 70%, 80%, 85%, 90% or 95%.

In the second aspect of the present invention, it provides a method for preparing a cuboidal cyclodextrin framework-RGD composition, which comprises the steps of:
(1) providing a cuboidal cyclodextrin-organometallic framework (CD-MOF);
(2) crosslinking step: crosslinking the cuboidal cyclodextrin-metal organic framework of step (1) with a crosslinking agent to obtain a cyclodextrin framework (COF);
(3) RGD modification step, modifying RGD onto the cyclodextrin framework of step (2), thereby obtaining the cuboidal cyclodextrin framework-RGD composition (RGD-COF).

In another preferred embodiment, in step (2), the cross-linking is to cross-link the hydroxyl group (-OH) on the cuboidal cyclodextrin-metal organic framework by the cross-linking agent.

In another preferred embodiment, in step (2), the cyclodextrin framework is a cyclodextrin framework with water stability.

In another preferred embodiment, in step (3), RGD is modified on the surface of the cyclodextrin framework of step (2), thereby obtaining the cuboidal cyclodextrin framework-RGD composition.

In another preferred embodiment, the step (2) further comprises:
(2a) optionally, a step of retaining the cubic shape of the COF;
(2b) optionally, a step of removing metal ions.

In another preferred embodiment, the cross-linking step comprises the following sub-steps:
(2a) a dispersing step: dispersing the cuboidal cyclodextrin-metal organic framework in an organic solvent A to obtain a dispersing liquid 2a;
(2b) a step of adding crosslinking agent and the catalyst: under a crosslinking reaction temperature T, adding a crosslinking agent and a catalyst A into the dispersion liquid 2a, and obtaining a dispersion liquid 2b after a reaction time t1;
(2c) optionally, a cooling step: cooling the dispersion liquid 2b to obtain a cooled dispersion liquid 2b;
(2d) optionally, a step of terminated the reaction: adding a reaction terminator into the cooled dispersion 2b to obtain a dispersion 2d;
(2e) optionally, a centrifugal step: centrifuging the dispersion liquid 2d to obtain a crystal 2e;
(2f) optionally, a washing step: washing the crystal 2e to obtain a washed crystal 2f;
(2g) optionally, a drying step: drying the washed crystal 2f; and
(2h) obtaining the cuboidal cyclodextrin framework (COF).

In another preferred embodiment, the dispersion 2a is a suspension, an emulsion, a suspension or a colloid.

In another preferred embodiment, the dispersion 2b is a suspension, an emulsion, a suspension or a colloid.

In another preferred embodiment, the dispersion 2d is a suspension, an emulsion, a suspension or a colloid.

In another preferred embodiment, the temperature T of the crosslinking reaction is 30-110°C, preferably 40-100 °C, more preferably 60-90 °C, and most preferably 70-80 °C.

In another preferred embodiment, the temperature T of the crosslinking reaction is 80°C, 40°C, 100°C, 50°C, 70°C, or 60°C

In another preferred embodiment, the molar ratio of the cyclodextrin-organometallic framework of step (2a) to the crosslinking agent of step (2b) is 1: 1-1:20, preferably 1: 2-1:10, and more preferably 1: 4-1: 8.

In another preferred embodiment, the molar ratio of the cyclodextrin-organometallic framework of step (2a) to the crosslinking agent of step (2b) is 1: 6, 1: 2, 1: 5, 1:10, 1:20, 1: 8, 1:15, or 1: 4.

In another preferred embodiment, in step (2b), the crosslinking agent and the catalyst are added under stirring at a stirring speed of 200-1000 rpm, preferably 300-800 rpm, and more preferably 400-600 rpm.

In another preferred embodiment, in step (2b), the crosslinking agent and the catalyst are added under stirring at a stirring speed of 600 rpm, 200 rpm, 1000 rpm, 900 rpm, 700 rpm, 400 rpm, 500 rpm, or 700 rpm.

In another preferred embodiment, in step (2b), the hydroxyl groups in the cyclodextrin-organometallic framework are cross-linked by covalent bonds.

In another preferred embodiment, in step (2b), the reaction time t1 is 4-48h, preferably 8-24h, and more preferably 12-16h.

In another preferred embodiment, in step (2b), the reaction time t1 is 24 h, 48 h, 4 h, 12 h, 8 h, or 16 h.

In another preferred embodiment, in step (2c), the cooling is to cool to room temperature.

In another preferred embodiment, in step (2d), the reaction terminator is ethanol.

In another preferred embodiment, in step (2d), the reaction terminator is 95-100% ethanol or 70-90% ethanol.

In another preferred embodiment, in step (2e), the centrifugation is conducted at 3000-4500 rpm for 3-15 min, and preferably at 4000 rpm for 5 min.

In another preferred embodiment, in step (2f), the washing is to wash by using ethanol, water and/or acetone.

In another preferred embodiment, the COF is in a cubic shape.

In another preferred embodiment, the particle size of the COF is 50 nm to 50 µm, and preferably 50-500 nm or 1-50 µm.

In another preferred embodiment, the particle size of the COF is 100-500 nm.

In another preferred embodiment, the particle size of the COF is 100-300 nm, and preferably 150-200 nm.

In another preferred embodiment, the particle size of the COF is 1-50 µm, preferably 30-50 µm, and more preferably 10-30 µm.

In another preferred embodiment, the particle size of the COF is 1-5 µm.

In another preferred embodiment, the particle size of the COF is 200-500 nm, 100-300 nm, 200-400 nm, 200-500 nm, 1-10 µm, 1-5 µm, 30-50 µm, or 10-30 µm.

In another preferred embodiment, the RGD modification step comprises the following sub-steps:
(3a) a dispersing step: dispersing the cuboidal cyclodextrin framework (COF) and RGD in an organic solvent B to obtain a dispersing liquid 3a;
(3b) conjugating step: adding a catalyst B into the dispersion liquid 3a to conjugate the cuboidal cyclodextrin framework with RGD, wherein the reaction time is t2;
(3c) optionally, a centrifugation step;
(3d) optionally, a washing step;
(3f) optionally, a drying step;
(3g) obtaining the cuboidal cyclodextrin framework-RGD composition (RGD-COF).

In another preferred embodiment, in step (3b), the conjugating is to conjugate the hydroxyl groups on the surface of the cuboidal cyclodextrin framework with the carboxyl groups of the RGD.

In another preferred embodiment, the dispersion 3a is a uniformly mixed dispersion.

In another preferred embodiment, the dispersion 3a is a suspension, an emulsion, a suspension, or a colloid.

In another preferred embodiment, the conjugating step is carried out under the condition of heating and stirring.

In another preferred embodiment, a magnetic stirrer rotates at 200-1000 rpm during the stirring process.

In another preferred embodiment, the magnetic stirrer rotates at 200 rpm, 400 rpm, 500 rpm, 600 rpm, 700 rpm, 900 rpm, or 1000 rpm during the stirring process.

In another preferred embodiment, the heating temperature is 20-40 °C, and preferably 37-38 °C.

In another preferred embodiment, the heating temperature is 20°C, 25°C, 30°C, 35°C, 37°C, or 40°C.

In another preferred embodiment, the reaction time t2 is 4-48 h, preferably 8-24 h, and more preferably 12-20 h.

In another preferred embodiment, the reaction time t2 is 4h, 6h, 8h, 12h, 18h, 24h, or 48h.

In another preferred embodiment, in step (3a), wherein the molar ratio of COF to RGD is 1: 0.1 to 1:10, preferably 1: 0.2 to 1: 5, and preferably 1: 1.

In another preferred embodiment, in step (3a), the molar ratio of COF to RGD is 1: 1, 1: 2, 5: 1, 4: 1, 2: 1, 1: 3 or 1: 5.

In another preferred embodiment, in step (3g), the cuboidal cyclodextrin framework-RGD composition (RGD-COF) has a particle size of 200-500 nm, 100-300 nm, 200-400 nm, 200-500 nm, 1-10 µm, 1-5 µm, 30-50 µm, or 10-30 µm.

In another preferred embodiment, the crosslinking agent is selected from the group consisting of peroxides, polyisocyanates, glycidyl ethers, diacids or polyacids, dialdehydes or polyaldehydes, compounds containing carbonyl groups, epoxides, acrylates, acyl chlorides, and combinations thereof.

In another preferred embodiment, the peroxide is selected from the group consisting of benzoyl peroxide, dicumyl peroxide, tert-butyl peroxide, and combinations thereof.

In another preferred embodiment, the polyisocyanate is selected from the group consisting of isocyanates, toluene diisocyanates, diphenylmethane diisocyanates, dicyclohexylmethane diisocyanates, hexamethylene diisocyanates, lysine diisocyanates, and combinations thereof.

In another preferred embodiment, the glycidyl ether is selected from the group consisting of ethylene glycol diglycidyl ether, polypropylene glycidyl ether, trimethylolpropane triglycidyl ether, n-butyl glycidyl ether, and combinations thereof.

In another preferred embodiment, the diacid or polyacid is selected from the group consisting of citric acid, malonic acid, succinic acid, phthalic acid, isophthalic acid, and combinations thereof.

In another preferred embodiment, the dialdehydes or polyaldehydes are selected from the group consisting of glyoxal, glutaraldehyde, succinaldehyde, and combinations thereof.

In another preferred embodiment, the carbonyl-containing compound is selected from the group consisting of diphenyl carbonate, N,N'-carbonyldiimidazole, N,N'-dimethylimidazoline, dicyclohexylcarbodiimide, and combinations thereof.

In another preferred embodiment, the epoxide is selected from the group consisting of epichlorohydrin, propylene oxide, 1,4-dioxane, and combinations thereof.

In another preferred embodiment, the acrylate is selected from the group consisting of ethylene glycol dimethacrylate, hydroxyethyl acrylate, hydroxypropyl acrylate, methacrylic acid, hydroxyethyl methacrylate, hydroxypropyl methacrylate, and combinations thereof.

In another preferred embodiment, the acyl chloride is selected from the group consisting of succinyl chloride, tetraisocyanate, or a combination thereof.

In another preferred embodiment, the crosslinking agent is diphenyl carbonate.

In another preferred embodiment, the crosslinking agent is epichlorohydrin.

In another preferred embodiment, the catalyst A is selected from the group consisting of 4-dimethylaminopyridine, triethylamine, 1-(3-dimethylaminopropyl)-3-ethylcarbodiimide or salts thereof, N,N'-succinimide carbonate, N-hydroxysuccinimide, pyridine, and combinations thereof; and is preferably triethylamine;

The organic solvent A is selected from the group consisting of dimethylformamide, tetrahydrofuran, methanol, acetonitrile, acetone, isopropanol, ethyl acetate, chloroform, n-hexane, ethanol and dichloromethane.

In another preferred embodiment, the organic solvent A is selected from the group consisting of dimethylformamide, tetrahydrofuran, methanol, acetonitrile, acetone, isopropanol, ethyl acetate, chloroform, n-hexane, ethanol, dichloromethane, and combinations thereof.

In another preferred embodiment, the organic solvent A is dimethylformamide.

In another preferred embodiment, the catalyst A is selected from the group consisting of 4-dimethylaminopyridine, triethylamine, 1-(3-dimethylaminopropyl)-3-ethylcarbodiimide or salts thereof, N,N'-succinimide carbonate, N-hydroxysuccinimide, pyridine, and combinations thereof.

In another preferred embodiment, the catalyst A is triethylamine.

In another preferred embodiment, the organic solvent B is selected from the group consisting of dimethylformamide, tetrahydrofuran, methanol, acetonitrile, acetone, isopropanol, ethyl acetate, chloroform, n-hexane, ethanol, dichloromethane, and combinations thereof.

In another preferred embodiment, the organic solvent B is dimethylformamide.

In another preferred embodiment, the catalyst B is selected from the group consisting of 4-dimethylaminopyridine, triethylamine, 1-(3-dimethylaminopropyl)-3-ethylcarbodiimide or salts thereof, N-hydroxysuccinimide, N,N '-succinimide carbonate, pyridine, and combinations thereof.

In another preferred embodiment, the catalyst B is 4-dimethylaminopyridine.

In another preferred embodiment, the catalyst A is selected from the group consisting of 4-dimethylaminopyridine, triethylamine, 1-(3-dimethylaminopropyl)-3-ethylcarbodiimide or salts thereof, N,N '-succinimide carbonate, N-hydroxysuccinimide, pyridine, and combinations thereof; the catalyst B is selected from the group consisting of 4-dimethylaminopyridine, triethylamine, 1-(3-dimethylaminopropyl)-3-ethylcarbodiimide or salts thereof, N-hydroxysuccinimide, N,N'-succinimide carbonate, pyridine, and combinations thereof.

In another preferred embodiment, the organic solvent A is selected from the group consisting of dimethylformamide, tetrahydrofuran, methanol, acetonitrile, acetone, isopropanol, ethyl acetate, chloroform, n-hexane, and combinations thereof; and
the organic solvent B is selected from the group consisting of dimethylformamide, tetrahydrofuran, methanol, acetonitrile, acetone, isopropanol, ethyl acetate, chloroform, n-hexane, ethanol, dichloromethane, and combinations thereof.

In another preferred embodiment, the catalyst B is selected from the group consisting of 4-dimethylaminopyridine, triethylamine, 1-(3-dimethylaminopropyl)-3-ethylcarbodiimide or salts thereof, N-hydroxysuccinimide, N,N'-succinimide carbonate, pyridine, and combinations thereof; and
the organic solvent B is selected from the group consisting of dimethylformamide, tetrahydrofuran, methanol, acetonitrile, acetone, isopropanol, ethyl acetate, chloroform, n-hexane, ethanol, dichloromethane, and combinations thereof.

In another preferred embodiment, the organic solvent B is dimethylformamide.

In another preferred embodiment, the catalyst B is 4-dimethylaminopyridine.

In another preferred embodiment, the RGD sequence comprises linear RGD, GRGD, RGDS, GRGDS; annular RGD, GRGD, RGDS, GRGDS, and preferably linear RGD sequence, including GRGD, RGDS, GRGDS.

In the third aspect of the present invention, it provides a cuboidal cyclodextrin framework-RGD composition loaded with a drug selected from the group consisting of an antibacterial drug, a hemostatic drug, an antithrombotic drug, an anti-infective drug, or a combination thereof, wherein the cuboidal cyclodextrin framework-RGD composition is the composition of the first aspect of the present invention or is prepared by the method of the second aspect of the present invention.

In another preferred embodiment, the antibacterial agent is selected from the group consisting of silver nanoparticle, penicillin, cephalosporin, minocycline, doxycycline, tetracycline, chloramphenicol, lincomycin, vancomycin, and combinations thereof.

In another preferred embodiment, the hemostatic drug is selected from the group consisting of tranexamic acid, aminocaproic acid, vitamin K1, and combination thereof.

In another preferred embodiment, the antithrombotic drug is selected from the group consisting of aspirin, clopidogrel, ticlopidine, cilostazol, tirofiban, ozagrel, rivaroxaban, and combinations thereof.

In another preferred embodiment, the anti-infective agent is selected from the group consisting of sulfadiazine, ceftriaxone, amoxicillin, levofloxacin, and combinations thereof.

In another preferred embodiment, the drug-loaded cuboidal cyclodextrin framework-RGD composition is for intravenous injection (nanometer scale) and topical administration (micron scale).

In another preferred embodiment, the composition also has one or more of the following characteristics:
(1) the cyclodextrin framework-RGD composition has a particle size of 50 nm-50 µm, preferably 100-500 nm or 1-5µm;
(2) the drug loading of the composition is 1%-20%, preferably from 5%-10%.

In another preferred embodiment, the cyclodextrin framework-RGD composition has a particle size of 100-500 nm; or the cyclodextrin framework-RGD composition have a particle size of 1-5µm.

In another preferred embodiment, the drug loading of the composition is 2.1%-13.5%.

In another preferred embodiment, the drug loading of the composition is preferably 2.7%-8.6%.

In another preferred embodiment, the cyclodextrin framework-RGD composition is in a form of powder.

In another preferred embodiment, the cyclodextrin framework-RGD composition is in a form or particle.

In another preferred embodiment, the drug-loaded cuboidal cyclodextrin framework-RGD composition reduces clot formation time by more than 30%, 40%, 50%, 60%, 70%, 80% or 90%.

In another preferred embodiment, the drug-loaded cuboidal cyclodextrin framework-RGD composition reduces bleeding time by more than 50%, 60%, 70%, 80%, 85%, 90% or 95%.

In another preferred embodiment, the drug-loaded cuboidal cyclodextrin framework-RGD composition reduces blood loss by more than 50%, 60%, 70%, 80%, 85%, 90% or 95%.

In a fourth aspect of the present invention, it provides a use of an active ingredient selected from the group consisting of:
(i) the cyclodextrin framework-RGD composition of the first aspect of the present invention;
(ii) the drug-loaded cuboidal cyclodextrin framework-RGD composition according to the third aspect of the present invention;
(iii) a cyclodextrin framework (COF);
(iv) any combination of (i), (ii) or (iii); wherein the active ingredient is used for:
   (a) preparation of a drug-loading material;
   (b) preparation of a therapeutic and/or diagnostic reagent or kit;
   (c) preparation of a hemostatic drug and/or material;
   (d) preparation of an anti-infective drug and/or material;
   (e) preparation of an antimicrobial agent and/or material;
   (f) preparation of a medicament and/or material for promoting wound healing; or
   (g) preparation of a medicament and/or material for prevention and/or treatment of thrombosis.

In another preferred embodiment, the therapeutic and/or diagnostic reagent or kit is used for disease treatment and/or diagnosis.

In another preferred embodiment, the disease is selected from the following group: thrombosis, atherosclerosis, stroke, tumor, hemorrhage, inflammation, and infection.

In another preferred embodiment, the diagnostic reagent or kit is used for medical CT development.

In another preferred embodiment, the therapeutic and/or diagnostic reagent or kit is used for anti-tumor, hemostatic, anti-inflammatory, or anti-infection.

In another preferred embodiment, the hemostatic drug and/or material can reduce the clot formation time by more than 30%, 40%, 50%, 60%, 70%, 80% or 90%.

In another preferred embodiment, the hemostatic drug and/or material can shorten the bleeding time by more than 50%, 60%, 70%, 80%, 85%, 90% or 95%.

In another preferred embodiment, the hemostatic drug and/or material can reduce blood loss by more than 50%, 60%, 70%, 80%, 85%, 90% or 95%.

In a fifth aspect of the present invention, it provides a pharmaceutical composition comprising:
(1) an active ingredient which is a cyclodextrin framework-RGD composition of the first aspect of the present invention or a drug-loaded cuboidal cyclodextrin framework-RGD composition of the third aspect of the present invention; and
(2) a pharmaceutically acceptable carrier.

In another preferred embodiment, the pharmaceutical composition is a capsule, a tablet or a granule.

In another preferred embodiment, the carrier is selected from the group consisting of diluents, excipients, fillers, binders, wetting agents, disintegrants, absorption enhancers, surfactants, adsorption carriers, lubricants, and combinations thereof.

In another preferred embodiment, the pharmaceutical composition is formulated in a solid dosage form or a liquid dosage form, and is preferably suitable for oral administration, and more preferably suitable for injection administration.

In another preferred embodiment, the solid dosage forms include capsules, tablets, pills, powders and granules.

In another preferred embodiment, the liquid dosage form comprises a pharmaceutically acceptable emulsion, solution, suspension, syrup or tincture.

In another preferred embodiment, the pharmaceutical composition is a capsule, a tablet, a granule, or an injection.

In another preferred embodiment, the pharmaceutical composition further comprises a surfactant, which is selected from the group consisting of polysorbitan-80, polysorbitan-60, polyethylene glycol glycerol fatty acid esters, sorbitol fatty acid esters and mixtures of two or more.

In another preferred embodiment, the pharmaceutical composition reduces the clot formation time by more than 30%, 40%, 50%, 60%, 70%, 80% or 90%.

In another preferred embodiment, the pharmaceutical composition reduces the bleeding time by more than 50%, 60%, 70%, 80%, 85%, 90% or 95%.

In another preferred embodiment, the pharmaceutical composition reduces blood loss by more than 50%, 60%, 70%, 80%, 85%, 90% or 95%.

It should be understood that within the scope of the present invention, the above-described technical features of the present invention and the technical features described in detail hereinafter (e.g., in examples) may be combined with each other to constitute a new or preferred technical solution, which is not redundantly repeated one by one herein due to limitation of space.

### Description of the drawings

Fig. 1 is a scanning electron microscope image of nano CD-MOF in Example 1.
Fig. 2 is a scanning electron microscope image of nano COF in Example 1.
Fig. 3 is a scanning electron microscope image of nano RGD-COF in Example 1.
Fig. 4 is a dynamic light scattering particle size distribution diagram of nano RGD-COF in Example 1.
Fig. 5 shows the physical stability of nano-RGD-COF in Example 1 (■: water; ●: normal saline; ▲: PBS pH 7.4; ◆: rat plasma).
Fig. 6 shows the cytotoxicity of nano-RGD-COF in Example 1.
Fig. 7 is a scanning electron microscope image of micron CD-MOF in Example 6.
Fig. 8 is a scanning electron microscope image of micron COF in Example 6.
Fig. 9 is a scanning electron microscope image of micron RGD-COF in Example 6.
Fig. 10 is a scanning electron microscope image of spherical RGD-NS in Example 9.
Fig. 11 shows clot formation time *in vitro* for RGD-COF in Example 10.
Fig. 12 shows that RGD-COF in Example 11 can significantly reduce the bleeding time *in vivo* in mouse tail transection model.
Fig. 13 shows that RGD-COF in Example 11 can significantly reduce blood loss *in vivo* in the mouse tail transection model.
Fig. 14 shows the time-antimicrobial curve of silver-loaded RGD-COF and the like in Example 12.
Fig. 15 shows the healing effect of silver-loaded RGD-COF and the like in Example 12 on rat wounds.
Fig. 16 shows that RGD-COF in Example 16 can target mesenteric thrombus *in vivo,* and has high co-localization with activated platelets at thrombus site.

### Detailed Description of the Invention

After extensive and intensive research, the inventors have developed a cuboidal cyclodextrin framework-RGD composition (RGD-COF) and its preparation method therefor. The cuboidal cyclodextrin framework-RGD composition of the present invention comprises a cyclodextrin framework with a cubic structure and RGDs, which can evade phagocytosis and clearance of macrophages, enhance migration and adhesion to injured blood vessels, efficiently target and aggregate activated platelets at injured blood vessels, and realize targeted therapy of blood vessel-related diseases (such as hemorrhage and thrombosis). Taking advantage of the controllable size of CD-MOF, a new and efficient carrier material for intravenous injection (nanoscale) or external application (micron-scale) was obtained by crosslinking and RGD modifying nanoscale and micron-scale CD-MOF as basic materials. Specifically, the nanoscale cuboidal cyclodextrin framework-RGD composition of the present invention can reduce the bleeding time and blood loss by 90% in the mouse tail transection model by intravenous injection; micron-scale cyclodextrin framework-RGD composition can reduce bleeding time by 60%in rat femoral artery injury model by topically external application, thereby greatly improving hemostatic efficiency. The present invention has been completed on this basis.

### Term

### Metal organic framework material

Metal organic framework (MOF) is an inorganic-organic hybrid material formed by self-assembly of metals (metal ions, metal ion clusters or metal chains) and organic bridging ligands in the form of coordination bonds under mild conditions. Due to the high porosity and large specific surface area of MOFs, and there are many combination of different inorganic and organic components, so that the structure and composition of MOFs are diverse, which provides a new research direction for the application of MOFs in gas storage, adsorption and separation, catalysis, drug delivery and other fields.

### Cyclodextrin

Cyclodextrin (CD) is the general name of a series of cyclic oligosaccharides produced by amylose under the action of glucosyltransferase, and usually contains 6 to 12 D-glucopyranose units. Among them, the molecules containing 6, 7 and 8 glucose units, which are called as α, β-and γ-cyclodextrins respectively, have been studied more and have important practical significance. Cyclodextrin is an ideal host molecule similar to enzyme, and has the characteristics of enzyme model by itself.

### Cyclodextrin-organometallic framework

As used herein, the terms "cyclodextrin-organometallic framework" and "CD-MOF" are used interchangeably, and refer to a new, safe and medicinal cuboidal cyclodextrin-organometallic framework, namely CD-MOF, which is formed by using cyclodextrin as organic ligand and metal ion as inorganic metal center.

Typically, the cyclodextrin-metal organic framework is a framework material formed by cyclodextrin and alkali metal salt; wherein the alkali metal includes but is not limited to Li⁺, K⁺, Rb⁺, Cs⁺, Na⁺, Mg²⁺, Cd²⁺, Sn²⁺, Ag⁺, Yb⁺, Ba²⁺, Sr²⁺, Ca²⁺, Pb²⁺, La³⁺, and preferably K⁺.

Typically, the cyclodextrin-organometallic framework material has an average particle size of 50 nm-50 microns, preferably 100-500 nm (nanoscale) or 1-5 microns (micron-scale).

Typically, a cyclodextrin-organometallic framework (CD-MOF) is prepared (refer to patent 201610125456.X) by a preparation method comprising the following steps: mixing a metal salt solution and a cyclodextrin aqueous solution, pre-adding a part of an organic solvent, reacting for a certain time through a solvent vapor diffusion method at a certain temperature, and then adding a size regulator to obtain the metal organic framework material based on cyclodextrin; or mixing a metal salt solution and a cyclodextrin aqueous solution, pre-adding a part of an organic solvent, shaking the reaction medium via solvothermal, microwave, ultrasonic wave to react the reactants quickly, and after a certain time of reaction, adding a size regulator to obtain the metal organic framework material based on cyclodextrin.

Typically, the concentration of the metal salt in the metal salt solution is 0.05-0.4 M, and preferably 0.2 M.

Typically, the concentration of cyclodextrin in the cyclodextrin aqueous solution is 0.013-0.05 M, and preferably 0.025 M.

Typically, the cyclodextrin is selected from the group consisting of: α-cyclodextrin, β-cyclodextrin, γ-cyclodextrin, hydroxypropyl-β-cyclodextrin, sulfobutyl-β-cyclodextrin, methyl-β-cyclodextrin, carboxymethyl-β-cyclodextrin, and combinations thereof.

In a preferred embodiment, the cyclodextrin is γ-cyclodextrin.

In a preferred embodiment, the cyclodextrin-organometallic framework is cuboidal.

In a preferred embodiment, the preparation of the cyclodextrin-organometallic framework comprises the steps of:
(1a) providing a first mixed solution, which comprises metal ions and cyclodextrins;
(2a) adding a first organic solvent into the first mixed solution to obtain a second mixed solution,
   wherein the volume ratio of the first organic solvent to the first mixed solution is (0.01-0.5): 1, preferably (0.03-0.3): 1, and more preferably (0.05-0.2): 1;
(3a) pretreating the second mixed solution to obtain a pretreated first mixture, wherein the pretreatment is selected from the group consisting of:
   (3a1) solvothermal evaporation treatment;
   (3b1) a combination of a solvothermal evaporation treatment and any treatment selected from group A, wherein the group A includes solvothermal treatment, microwave treatment, ultrasonic treatment, or a combination thereof;
(4a) separating and obtaining the precipitated cyclodextrin-organometallic framework material from the first mixture when the cyclodextrin-organometallic framework material is precipitated from the first mixture;
   or separating part or all of the solution from the first mixture as a third mixed solution; adding a second organic solvent and/or a size regulator into the third mixed solution, thereby precipitating a cyclodextrin-metal organic framework material; and
(5a) optionally separating and/or drying the cyclodextrin-organometallic framework material precipitate obtained in step (4a).

In another preferred embodiment, in step (3a), the solvothermal evaporation treatment comprises the steps of:
(i) placing the mixed solution into an open container I;
(ii) providing an open container II containing an organic solvent, placing the open container I and the open container II together in a closed system; and
(iii) heating/incubating the organic solvent in the open container II so that the organic solvent evaporates and diffuses into the mixed solution.

In another preferred embodiment, in step (iii), the closed system is subjected to a heat treatment integrally, so as to heat the organic solvent in the open container II.

In another preferred embodiment, in step (iii), the heating treatment comprises heating in water bath or oil bath.

In another preferred embodiment, in step (iii), the temperature of the heat treatment is 25-100°C, preferably 30-80°C, and more preferably 40-60°C.

In another preferred embodiment, in step (iii), the time of the heat treatment is 4-48 h, and preferably 6-24 h.

### Cyclodextrin framework material

The terms "cyclodextrin framework material", "cyclodextrin framework", "cubic cyclodextrin framework", "cuboidal cyclodextrin framework", "COF" are used interchangeably.

CD-MOF comprises K⁺ , can not be injected intravenously directly, and will disintegrate rapidly in aqueous environment, thus the stability of porous crystal structure before reaching the target site can not be ensured. In order to increase the stability of CD-MOF in water, only three strategies have been reported so far. Furukawa *et al.* crosslinked γ-CD-MOF with ethylene glycol diglycidyl ether to produce γ-CD-MOF hydrogels. However, this cross-linking reaction is extremely time-consuming, takes more than three days at 65°C and requires many steps to remove unreacted impurities. Li *et al.* doped fullerene (C60) into the hydrophobic cavity of γ-CD to improve the water stability of γ-CD-MOF. However, due to the weak interaction between ligand and cyclodextrin, the supramolecular assembly system can only maintain the structural integrity in a short time, and degrades after 24 hours in water. In addition, the occupation of γ-CD cavity by C60 may also reduce the drug loading capacity of γ-CD-MOFs. In summary, the synthesis of stable porous CD-MOF materials is still a huge challenge.

Therefore, in the present invention, diphenyl carbonate, epichlorohydrin and the like are used as crosslinking agent to cross-link the ordered CD in the CD-MOF, thereby successfully preparing cyclodextrin framework (COF) with water stability, which still retains the cube shape of the CD-MOF and can remove the coordinated K⁺. Moreover, the COF obtained by cross-linking the nanoscale CD-MOF can be used as a new nano-carrier for intravenous injection.

In a preferred embodiment, the inventors used diphenyl carbonate, epichlorohydrin, and the like as crosslinking agents to successfully crosslink CD in CD-MOF. Using triethylamine and pyridine as catalysts, a simple synthetic route, which can synthesize COF with good water stability within 4 hours, has been invented.

In a preferred embodiment, the preparation method of the cyclodextrin framework (COF) is as follows: a proper amount of prepared CD-MOFs powder is weighed and added into a round bottom flask which is fixed on the magnetic stirrer; a certain volume of organic solvent is added under the condition of heating and stirring; and a certain amount of crosslinking agent and catalyst are added to react for a period of time, so that the hydroxyl groups (-OH) in CD-MOF are crosslinked by covalent bonds; after the mixture is cooled to room temperature, the reaction was terminated by 95% ethanol; the precipitate is obtained by centrifuge, washed twice with 50% ethanol, water and acetone, and then dried in vacuum to obtain cyclodextrin framework (COF) with water stability.

### RGD

RGD sequence is Arg-Gly-Asp sequence. Endogenous RGD sequence exists in a variety of adhesion proteins (such as fibrinogen, vitronectin, fibronectin, von Willebrand factor), and plays a variety of biological functions. The specific binding between RGD sequence in α chain of fibrinogen A and GPIIb/IIIa receptor on activated platelet surface is the common pathway of platelet aggregation and thrombus formation. RGD sequence, as a specific sequence in fibrinogen, is an active site for fibrinogen to bind to activated GPIIb/IIIa receptor.

The RGD sequence of the invention comprises linear RGD, linear GRGD, linear RGDS, linear RGDV, linear RGDF, linear GRGDV, linear GRGDF, linear GRGDS, linear RGDDSP and linear RGDDAP; annular RGD, annular GRGD, annular RGDS, annular RGDV, annular RGDF, annular GRGDV, annular GRGDF, annular GRGDS, annular RGDDSP, and annular RGDDAP.

Typically, the RGD sequence of the present invention is preferably linear RGD sequence, including linear GRGD, linear RGDS, linear RGDF, linear GRGDS.

### Cuboidal cyclodextrin framework-RGD composition

As used herein, the terms "cuboidal cyclodextrin framework-RGD composition", "RGD modified COF", "RGD-COF", "cuboidal cyclodextrin framework-RGD polypeptide composition" are used interchangeably and refer to the cuboidal or cubic cyclodextrin framework-RGD composition according to the first aspect of the present invention.

Typically, the present invention provides a cuboidal cyclodextrin framework-RGD composition, whose structure is characterized in that the hydroxyl group (-OH) in the inside of cyclodextrin-organometallic framework material having cubic crystal structure is covalently connected by a suitable crosslinking agent to form a cuboidal cyclodextrin framework with water stability, while in the outside, the hydroxyl group (-OH) on the surface is covalently connected with the carboxyl group (-COOH) of RGD by an activator to carry out biological modification on the cyclodextrin framework. Cuboidal cyclodextrin framework is used as artificial platelet carrier and RGD is used as target warhead, which specifically binds to GPIIb/IIIa receptor on activated platelet surface and targets activated platelets at vascular injury site. RGD modified cyclodextrin framework material is useful as a new high-efficiency artificial platelet, which can improve the migration ability of artificial platelets to injured blood vessels, improve the targeting efficiency to bleeding sites, and promote the binding with activated platelets.

Specifically, the invention provides a cuboidal cyclodextrin framework-RGD composition composed of COF and RGD, wherein the interior thereof is a cyclodextrin framework with cubic crystal structure, and the surface thereof is biologically modified by RGD. The cuboidal cyclodextrin framework is used as a carrier and RGD is used as target warhead to target GPIIb/IIIa receptor on the surface of activated platelet at vascular injury site. RGD-modified COF is a novel and highly effective functional material, which can improve the migration ability to injured blood vessels, improve the targeting efficiency to injured sites, and promote the binding and aggregation with activated platelets.

The cuboidal cyclodextrin framework-RGD composition of the invention is different from the reported artificial platelets. The Anirban Sen Gupta team have adopted RGD and collagen binding peptide to modify spherical liposomes, and the constructed artificial platelets have certain hemostatic effect in the mouse tail transection model after being injected through tail vein, but the targeting and hemostatic effect still need to be improved. Tan Yingxia *et al.* have invented an artificial platelet PLGA-PEG-RGD by using PLGA-PEG nanoparticles loaded with RGD. PLGA-PEG-RGD is regular spherical, but its particle size is uneven. Although PLGA-PEG-RGD can be used as systemic nano-hemostatic material for intravenous use, its hemostatic effect in rat liver injury model is limited, and its bleeding time can only be reduced by about 30%.

The nanoscale cuboidal cyclodextrin framework-RGD composition of the present invention can reduce the bleeding time and blood loss by 90% in mouse tail transection model by intravenous injection; micron-scale cyclodextrin framework-RGD composition can reduce bleeding time by 60% and greatly improve hemostatic efficiency in rat femoral artery injury model by topically application.

In a preferred embodiment, the preparation method of the cuboidal cyclodextrin framework-RGD composition (RGD-COF) comprises the following steps: weighing and adding a proper amount of COF and RGD into a round bottom flask, adding a certain volume of organic solvent B, mixing evenly, adding a proper amount of catalyst B and activator, heating and stirring on a magnetic stirrer for a proper time, so that the hydroxyl groups (-OH) on the surface of COF and the carboxyl groups (-COOH) of RGD are sufficiently conjugated. After completion of the reaction, the product is washed with equal volume of anhydrous DMF and water twice, freeze-dried overnight to give the cuboidal cyclodextrin framework-RGD composition (abbreviated as RGD-COF) , which is then frozen at -20°C for use.

The cuboidal cyclodextrin framework-RGD composition (RGD-COF) provided by the present invention has a high-efficiency targeting performance, can be used for systemic blood loss and thrombosis by intravenous injection, and can also be used for treating trauma by local medication, thus providing more choices for the treatment of complex war trauma, accidental trauma, surgical bleeding, stroke and tumor-related diseases, and has broad application prospects.

### Preparation method of cuboidal cyclodextrin framework-RGD composition

The invention provides a preparation method of the cuboidal cyclodextrin framework-RGD composition.

In a preferred embodiment, a pharmaceutically acceptable cyclodextrin is used as an organic linker, and K⁺ is used as inorganic metal center, to prepare CD-MOF with cubic morphology and uniform particle size. CD of CD-MOFs is crosslinked by crosslinking agent to prepare COF with water stability. Carboxyl group (-COOH) of RGD sequence is bound to COF surface by ester bond to form cuboidal cyclodextrin framework-RGD composition (RGD-COF). The CD-MOF of the present invention is prepared based on a method for preparing a cyclodextrin-metal organic framework material (see CN 201610125456.X), which is fast, simple, adjustable in size and high in yield.

In another preferred embodiment, the method for preparing the cuboidal cyclodextrin framework-RGD composition (RGD-COF) comprises the steps of:
(1) preparing cuboidal cyclodextrin-organometallic framework (CD-MOF);
(2) crosslinking the cuboidal cyclodextrin-metal organic framework (CD-MOF) by using crosslinking agent, removing metal ions, and retaining the cubic shape, thereby obtaining a cyclodextrin framework (COF);
   wherein preferably, the specific crosslinking reaction process comprises: dispersing the cubic cyclodextrin-organometallic framework in an organic solvent A, adding crosslinking agent and catalyst A under stirring condition at a certain temperature to react, so that hydroxyl groups in cyclodextrin-organometallic framework are crosslinked through covalent bonds; cooling, quenching the reaction with ethanol, centrifuging, washing and drying to obtain the cubic cyclodextrin framework (COF);
(3) modifying RGD on the surface of the cuboidal cyclodextrin framework to obtain the cuboidal cyclodextrin framework-RGD composition (RGD-COF). Preferably, the specific RGD modification process comprises the following steps: adding the cuboidal cyclodextrin framework and RGD into an organic solvent B in a certain proportion, mixing evenly, adding a catalyst B, heating and stirring, so that the hydroxyl groups on the surface of the cuboidal cyclodextrin framework and the carboxyl groups of the RGD are sufficiently conjugated, and washing and drying after the reaction is completed, thereby obtaining a cuboidal cyclodextrin framework-RGD composition (RGD-COF).

### Pharmaceutically acceptable carrier

"Pharmaceutically acceptable carrier" refers to one or more compatible solid or liquid fillers or gels suitable for human use and of sufficient purity and low enough toxicity. "Compatibility" herein refers to the ability of components of a composition to blend with each other and with the compounds of the invention without significantly reducing the efficacy of the compounds. Some examples of pharmaceutically acceptable carriers are cellulose and its derivatives (such as sodium carboxymethyl cellulose, sodium ethyl cellulose, cellulose acetate, etc.), gelatin, talc, solid lubricants (such as stearic acid and magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween®), wetting agents (such as sodium dodecyl sulfate), colorants, flavoring agents, stabilizers, antioxidants, preservatives, pathogen-free hot water, and the like.

### The main advantages of the invention

The invention provides an intravenous (nanoscale) or topical (micron-scale) cuboidal cyclodextrin framework-RGD composition (RGD-COF) and the preparation method therefor, and the RGD-COF has a high safety and good biocompatibility. It can be used for targeted therapy of vascular-related diseases, including traumatic blood loss, deep hemorrhage, atherosclerosis, stroke, thrombosis and tumor, and has the following technical advantages:
(1) The RGD-COF of the invention has a regular cubic shape, breaks through the limitation of the spherical shape of the carrier in the past, effectively evades phagocytosis and clearance of macrophages, enhances the migration and adhesion to injured blood vessels, and improves targeting and treatment efficiency.
(2) After the modification with RGD sequence, the cuboidal RGD-COF can efficiently target and aggregate the activated platelets at the vascular injury site, realize the targeted treatment of vascular related diseases, and reduce the influence and side effects on the normal blood circulation system.
(3) The inventors fully utilized the advantages of controllable size of CD-MOF, take nano-level and micron-level CD-MOF as basic materials, and obtain a novel and efficient carrier material via crosslinking steps and RGD modification steps, which is suitable for intravenous injection and/or topical administration. Intravenous administration is suitable for treatment of trauma under complex conditions, and is suitable for the control of systemic blood loss and thrombotic diseases; while micron-scale RGD-COF is useful for hemostatic, anti-inflammatory and anti-infective treatment of external trauma such as car accident and operation.
(4) The RGD-COF of the present invention has a good stability, can be made into freeze-dried powder for use, is favorable for preservation, and is suitable for use under field conditions (such as equipping troops), and improves the treatment ability of complex war wounds.
(5) The preparation method of the present invention is simple and controllable, does not need any expensive equipment, can be produced on a large scale, does not have immunogenicity, and does not spread infectious diseases.
(6) The invention has the advantages of simple preparation method, high safety and good biocompatibility, and can be used for targeted diagnosis and treatment of uncontrolled hemorrhage such as traumatic blood loss, internal organ hemorrhage and deep hemorrhage, and thrombotic diseases.

The present invention will be further explained below in conjunction with specific examples. It should be understood that these examples are only used to illustrate the present invention and not to limit the scope of the present invention. The experimental methods that do not indicate specific conditions in the following examples usually follow the conventional conditions, such as those described in Sambrook and Russell et al., Molecular Cloning-A Laboratory Manual (3rd Edition) (2001) CSHL Press, or the conditions suggested by the manufacturer. Unless otherwise specified, percentages and parts are percentages by weight and parts by weight. The experimental materials and reagents used in the following examples are commercially available sources unless otherwise specified.

### Example 1

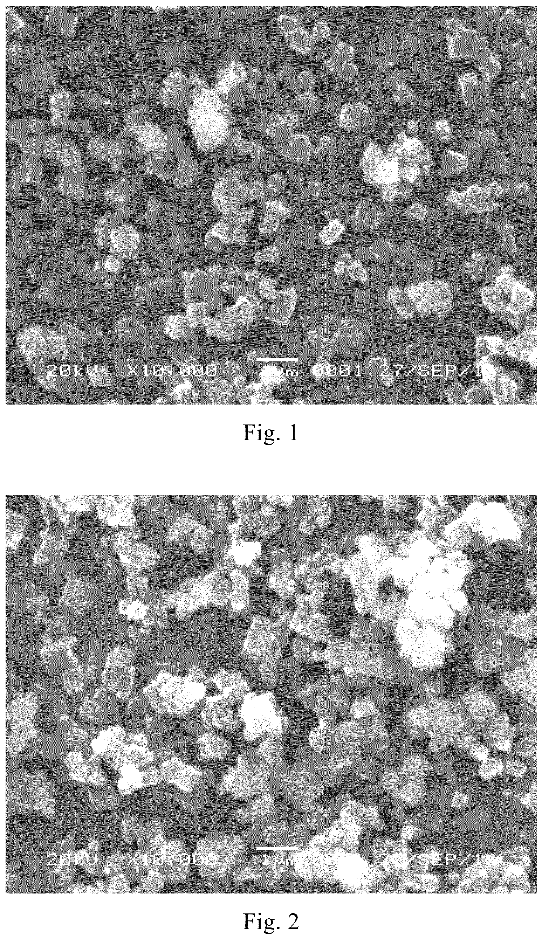

Preparation of nanoscale CD-MOF. By using solvothermal method, the mixture of γ-CD and KOH aqueous solution and a part of organic solvent was directly heated. A mixture of 163.0 mg γ-CDs and 56.0 mg KOH (molar ratio of γ-CD to KOH was 1: 8) was weighed and dissolved in 5 ml water, and 3 ml methanol was pre-added into the mixed solution, then heated in water bath at 50°C for 20 min. The solution was taken out, methanol of equal volume and 64 mg PEG 20000 were added, and after standing for 1 h, was centrifuged at 4000 rpm for 5 min. The precipitate was washed with ethanol (10 mLx2) and dichloromethane (10 mLx2), respectively, and the obtained crystal was dried in vacuum at 50°C for 12 h to give a nanoscale CD-MOF crystal (CD-MOF-Nano). The obtained CD-MOF had a regular cubic shape with a particle size of 200-500 nm (Fig. 1).

Preparation of nanoscale CD-MOF. 778.3 mg nanoscale CD-MOF powder was weighed and added into a round bottom flask which was fixed on a magnetic stirrer, and 10 mL dimethylformamide was added. The mixture was heated at 80°C and stirred at 600 rpm, and 771 mg of crosslinking agent diphenyl carbonate (the molar ratio of CD-MOF to crosslinking agent was 1: 6) and 450 µL of catalyst triethylamine were added. After 24 h, the reaction was cooled to room temperature. The reaction was terminated with 20 ml 95% ethanol, and the mixture was centrifuged at 4000 rpm for 5 min. The precipitate was washed with 50% ethanol (10 ml × 2), water (10 ml × 2) and acetone (10 ml × 2), respectively, and the obtained crystal was dried in vacuum at 50°C for 12 h to give a nanoscale CD-MOF with water stability, and the obtained COF had regular cubic shape with a particle size of 200-500 nm (Fig. 2).

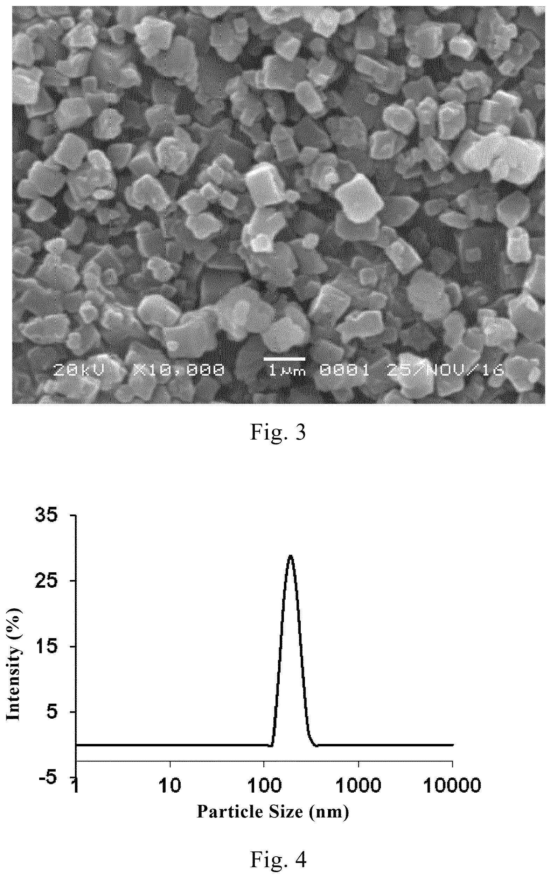

Preparation of nanoscale cyclodextrin framework-RGD composition (RGD-COF). 230 mg of nanoscale COF and 10 mg of linear GRGDS pentapeptide (molar ratio of COF to GRGDS was 1: 1) were weighed and added into a round bottom flask. 5 ml of dimethylformamide was added and stirred evenly, then 5 mg of 4-dimethylaminopyridine and 6 mg 1-(3-dimethylaminopropyl)-3-ethylcarbodiimide hydrochloride were added, and the mixture was stirred on a magnetic stirrer (600 rpm) at 37°C for 12h, so that the COF and GRGDS peptides were sufficiently conjugated. After completion of the reaction, the reaction mixture was centrifuged at 4000 rpm for 5 min, and the precipitate was washed with dimethylformamide (10 mLx2) and water (10 mLx2), freeze-dried at -50°C for 12 h to give a GRGDS modified COF. The results of SEM (Fig. 3) and DLS (Fig. 4) showed that the obtained RGD-COF had regular cubic shape with a particle size of 200-500 nm. The mass ratio of cyclodextrin framework material to RGD was 1: 0.049 (HPLC).

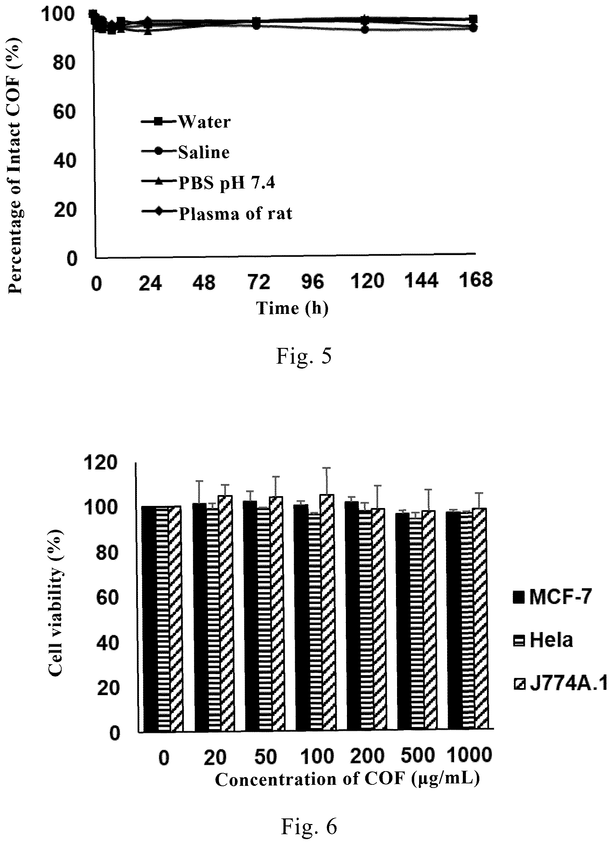

The obtained RGD-COF had good physical stability in water, phosphate buffer solution (PBS, pH = 7.4), normal saline or rat plasma (Fig. 5). MTT results showed that the RGD-COF had no cytotoxicity, and had good biocompatibility and safety (Fig. 6).

### Example 2

Nanoscale CD-MOF was prepared as in Example 1.

Preparation of nanoscale COF. 778.3 mg nanoscale CD-MOF powder was weighed and added into a round bottom flask which was fixed on a magnetic stirrer, and 10 mL acetonitrile was added. The mixture was heated at 40°C and stirred at 200 rpm. 194.6 mg of crosslinking agent N,N'-carbonyldiimidazole (the molar ratio of CD-MOF to crosslinking agent was 1: 2) and 450 µL of catalyst pyridine were added, and reacted for 48 h. The rest steps were the same as those in Example 1. The obtained COF had regular cubic shape with particle size of 100-300 nm.

Preparation of nanoscale RGD-COF. 230 mg nanoscale COF and 14 mg linear RGD (molar ratio of COF to RGD was 1: 2) were added into a round bottom flask, 5 ml acetonitrile was added and stirred evenly, and 5 mg N,N'-succinimide carbonate and 6 mg 1-(3-dimethylaminopropyl)-3-ethylcarbodiimide hydrochloride were added. The mixture was stirred on a magnetic stirrer (200 rpm) at 20°C for 48 h, and the rest steps were the same as those in Example 1. The obtained RGD-COF had regular cubic shape with particle size of 100-300 nm. The mass ratio of cyclodextrin framework to RGD measured by HPLC was 1: 0.08.

### Example 3

Nanoscale CD-MOF was prepared as in Example 1.

Preparation of nanoscale COF. 778.3 mg nanoscale CD-MOF powder was weighed and added in a round bottom flask which was fixed on a magnetic stirrer, and 10 mL tetrahydrofuran was added. The mixture was heated at 100°C and stirred at 1000 rpm, and 0.5 mL of crosslinking agent ethylene glycol diglycidyl ether (the molar ratio of CD-MOF to crosslinking agent was 1: 5) and 5 mg of catalyst N-hydroxysuccinimide were added, and reacted for 4 hours. The rest steps were the same as those in Example 1. The results of SEM and DLS showed that the obtained COF had regular cubic shape with particle size of 200-400 nm.

Preparation of nanoscale RGD-COF. 230 mg nanoscale COF and 3 mg cyclic GRGD polypeptide (the molar ratio of COF to cyclic GRGD was 5: 1) were weighed and added into a round bottom flask, 5 ml methanol was added and stirred evenly, and then 5 mg N-hydroxysuccinimide and 6 mg 1-(3-dimethylaminopropyl)-3-ethylcarbodiimide hydrochloride were added. The mixture was stirred on a magnetic stirrer (1000 rpm) at 40°C for 4 h, and the rest steps were the same as those in Example 1. The results of SEM and DLS showed that the obtained RGD-COF had regular cubic shape with a particle size of 200-400 nm. The mass ratio of cyclodextrin framework material to RGD measured by HPLC was 1: 0.005.

### Example 4

Nanoscale CD-MOF was prepared as in Example 1.

Preparation of nanoscale COF. 778.3 mg nanoscale CD-MOF powder was weighed and added into a round bottom flask which was fixed on a magnetic stirrer, and 10 mL acetone was added, heated at 50°C and stirred at 900 rpm. 0.6 mL glutaraldehyde (the molar ratio of CD-MOF to crosslinking agent was 1:10) and 5 mg pyridine catalyst were added, reacted for 12 h, and the rest steps were the same as those in Example 1. The results of SEM and DLS showed that the obtained COF had regular cubic shape with particle size of 200-500 nm.

Preparation of nanoscale RGD-COF. 200 mg nanoscale COF and 3 mg annular RGDS (molar ratio of COF to annular RGDS was 4: 1) were added into a round bottom flask, 5 ml acetone was added and stirred evenly, and then 5 mg N-hydroxysuccinimide and 6 mg 1-(3-dimethylaminopropyl)-3-ethylcarbodiimide hydrochloride were added. The mixture was stirred on a magnetic stirrer (900 rpm) at 40°C for 6 h, and the rest steps were the same as those in Example 1. The obtained RGD-COF had regular cubic shape with a size of 200-500 nm. The mass ratio of cyclodextrin framework material to RGD measured by HPLC was 1: 0.015.

### Example 5

Preparation of micron-scale CD-MOF. By Using the solvothermal method, the mixture of γ-CD and KOH aqueous solution and a part of organic solvent was directly heated. A mixture of 163.0 mg γ-CD and 56.0 mg KOH (molar ratio of γ-CD to KOH was 1: 8) was weighed and dissolved in 5 mL water, and 3 mL methanol was pre-added into the mixed solution, then heated in water bath at 50°C for 20 min. The solution was taken out, and then 64 mg PEG 2000 was added, and after standing for half an hour, centrifuged at 4000 rpm for 5 min. The precipitate was washed with ethanol (10 mL × 2) and dichloromethane (10 mL × 2), respectively, and the obtained crystals were dried in vacuum for 12 h to give the micron-sized CD-MOF crystals. The results of SEM and DLS showed that the obtained CD-MOF has a regular cubic shape with a size of 1-10 µm.

Preparation of micron-scale COF. 778.3 mg micron-scale CD-MOF powder was weighed and added into a round-bottom flask which was fixed on a magnetic stirrer, and 10 mL ethyl acetate was added, heated at 70°C and stirred at 700 rpm. 0.9 mL of crosslinking agent epichlorohydrin (the molar ratio of CD-MOF to crosslinking agent was 1:20) and 5 mg of catalyst 4-dimethylaminopyridine were added, reacted for 16 h, and the rest steps were the same as those in Example 1. The obtained COF had regular cubic shape with a particle size of 1-10 µm.

Preparation of micron-scale RGD-COF. 230 mg micron-scale COF and 4 mg RGDS (molar ratio of COF to RGDS was 2: 1) were added into a round bottom flask, 6 ml n-hexane was added and stirred evenly, and then 5 mg 4-dimethylaminopyridine and 0.2 ml triethylamine were added. The mixture was stirred on a magnetic stirrer (700 rpm) at 25°C for 24 h, and the rest steps were the same as those in Example 1 to give micron-scale cuboidal cyclodextrin framework-RGD composition (RGD-COF). The results of SEM and DLS showed that the obtained RGD-COF had regular cubic shape with a particle size of 1-10 µm, and the mass ratio of cyclodextrin framework material to RGD measured by HPLC was 1: 0.05.

### Example 6

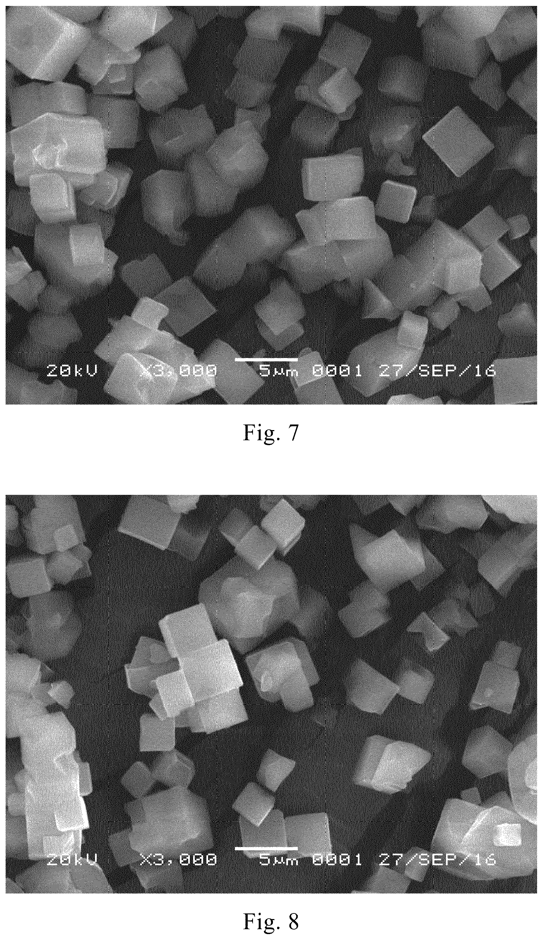

Micron-scale CD-MOF with a particle size of 1-5 µm was prepared as in Example 5 (Fig. 7).

Preparation of micron-scale COF. 778.3 mg micron-scale CD-MOF powder was weighed and added into a round-bottom flask which was fixed on a magnetic stirrer, and 10 mL acetone was added, heated at 50°C and stirred at 400 rpm. 0.7 mL of crosslinking agent toluene diisocyanate (the molar ratio of CD-MOF to crosslinking agent was 1: 8) and 450 µL of catalyst 1-(3-dimethylaminopropyl)-3-ethyl carbodiimide were added, reacted at 8 h, and the rest steps were the same as those in Example 1. The obtained COF had regular cubic shape with a particle size of 1-5 µm (Fig. 8).

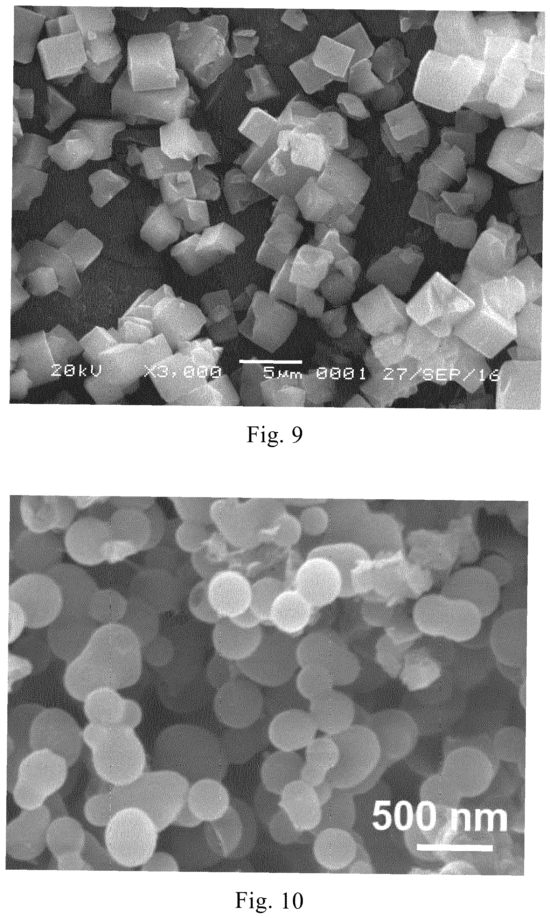

Preparation of micron-scale RGD-COF. 230 mg micron-scale COF and 18.5 mg annular RGD polypeptide (the molar ratio of COF to annular RGDS was 1: 2) were added in a round bottom flask, 6 ml isopropanol was added and stirred evenly, and then 5 mg 4-dimethylaminopyridine and 6 mg N-hydroxysuccinimide were added. The mixture was placed on a magnetic stirrer, stirred on a magnetic stirrer (400 rpm) at 35°C for 8 h, and the rest steps were the same as those in Example 1. The obtained RGD-COF had regular cubic shape with a particle size of 1-5 µm (Fig. 9), and the mass ratio of cyclodextrin framework material to RGD measured by HPLC was 1: 0.016.

### Example 7

Micron-scale CD-MOF was prepared as in Example 5.

Preparation of micron-scale COF. 778.3 mg micron-scale CD-MOF powder was weighed and added into a round bottom flask which was fixed on a magnetic stirrer, and 10 mL isopropanol was added. Under the condition of heating at 60°C and stirring at 500 rpm, 1729 mg of crosslinking agent citric acid (the molar ratio of CD-MOF to crosslinking agent was 1:15) and 450 µL of catalyst pyridine were added, reacted for 16 h, and the rest steps were the same as those in Example 1. The obtained COF had regular cubic shape with a particle size of 30-50 µm.

Preparation of micron-scale RGD-COF. 230 mg micron-scale COF and 30 mg GRGDS pentapeptide (molar ratio of COF to annular GRGDS was 1: 3) were weighed and added into a round bottom flask, 6 ml n-hexane was added and stirred evenly, and then 5 mg 4-dimethylaminopyridine and 6 mg N,N'-succinimidocarbonate were added. The mixture was stirred on a magnetic stirrer (500 rpm) at 30°C for 24 h, and the rest steps were the same as those in Example 1. The obtained RGD-COF had regular cubic shape with a particle size of 30-50 µm, and the mass ratio of cyclodextrin framework material to RGD measured by HPLC was 1: 0.065.

### Example 8

Micron-scale CD-MOF was prepared as in Example 5.

Preparation of micron-scale COF. 778.3 mg micron-scale CD-MOF powder was weighed and added into a round-bottom flask which was fixed on a magnetic stirrer, and 10 mL chloroform was added. Under the condition of heating at 70°C and stirring at 700 rpm, 403 mg of crosslinking agent succinyl chloride (the molar ratio of CD-MOF to crosslinking agent was 1: 4) and 450 µL of catalyst pyridine were added, reacted for 16 h, and the rest steps were the same as those in Example 1. The obtained COF had regular cubic shape with a particle size of 10-30 µm.

Preparation of micron-scale RGD-COF. 230 mg micron-scale COF and 25 mg annular GRGD (molar ratio of COF to annular GRGD was 1: 5) were added into a round bottom flask, 6 ml chloroform was added and stirred evenly, and then 5 mg 4-dimethylaminopyridine and 6 mg N-hydroxysuccinimide were added. The mixture was stirred on a magnetic stirrer (700 rpm) at 37°C for 18 h, and the rest steps were the same as those in Example 1. The results of SEM and DLS showed that the obtained RGD-COF had regular cubic shape with a particle size of 10-30 µm, and the mass ratio of cyclodextrin framework material to RGD measured by HPLC was 1: 0.46.

### Example 9

### Preparation of GRGDS modified spherical cyclodextrin nano-sponge (RGD-NS) (Comparative Example)

(1) Preparation of spherical cyclodextrin nano-sponge: 3.891 g γ-CD (3.000 mmol) was weighed and dissolved in 20 mL 0.1 M KOH aqueous solution by ultrasound, and then filtered with 0.8 µm filter membrane to obtain aqueous phase for later use. 1.297 g (8.00 mmol) of N,N'-carbonyldiimidazole was weighed and dissolved in 20 mL dichloromethane to obtain an organic phase. An aqueous solution of γ-CD and potassium hydroxide was added dropwise into the organic phase under continuous magnetic stirring (600 rpm). After reacting for 30 min, the precipitate was washed with deionized water and dry ethanol twice, centrifuged at 4000 rpm for 5min each time. The precipitate was collected, and freeze-dried to give the spherical cyclodextrin nano-sponge (CD-NS), and the obtained CD-NS was spherical with a particle size of 200-500 nm.
(2) GRGDS modified cyclodextrin nano-sponge. 230 mg spherical CD-NS and 10 mg GRGDS pentapeptide (the molar ratio of CD-NS to GRGDS was 1: 1) were added into a round bottom flask, 5 ml dimethylformamide was added and stirred evenly, and then 5 mg 4-dimethylaminopyridine and 6 mg 1-(3-dimethylaminopropyl)-3-ethylcarbodiimide hydrochloride were added. The mixture was stirred on a magnetic stirrer (600 rpm) at 37°C for 24 h, so that the CD-NS were sufficiently conjugated with the GRGDS peptide. After completion of the reaction, the mixture was centrifuged at 4000 rpm for 5 min, and precipitate was washed with dimethylformamide (10 mLx2) and water (10 mLx2), respectively, then freeze-dried at -50°C for 12 h to give a GRGDS modified CD-NS (abbreviated as RGD-NS). The results of SEM and DLS showed that GS5-NS had spherical with a particle size of 200-500 nm (Fig. 10). The mass ratio of cyclodextrin framework material to RGD measured by HPLC was 1: 0.052.

### Example 10

### Assay of coagulation function of RGD-COF in vitro

By measuring the clot formation time *in vitro,* the coagulation function of hemostatic materials *in vitro* was evaluated. Fresh whole blood of healthy rats anticoagulated with 3.2% sodium citrate was put into a clean test tube, and 80 µL CaCh (0.1 M) was added so that the final concentration of Ca²⁺ was 10 mM. Then the sample solution or normal saline was added until a final volume of the reaction system was 800 µL, and vortexed at 500 rpm/min for 10 s. Normal saline control group (blank control), RGD-COF group (20, 50, 100 µg/ml), COF group (100 µg/ml) and RGD-NS group (100 µg/ml) were set up. 60 µL of the whole blood treated as above was immediately taken into a 96-well plate with 12 wells per sample. Every 30s, one sample well was washed with normal saline to remove soluble blood components and prevent the coagulation reaction of the well until the washing solution was colorless, indicating that the soluble blood components had been completely removed. A stable blood clot was considered to have been formed when the clot covered the entire bottom of the well and the size of the clot was unchanged when washing at subsequent time points, and this time was recorded as the blood clot formation time *in vitro.*

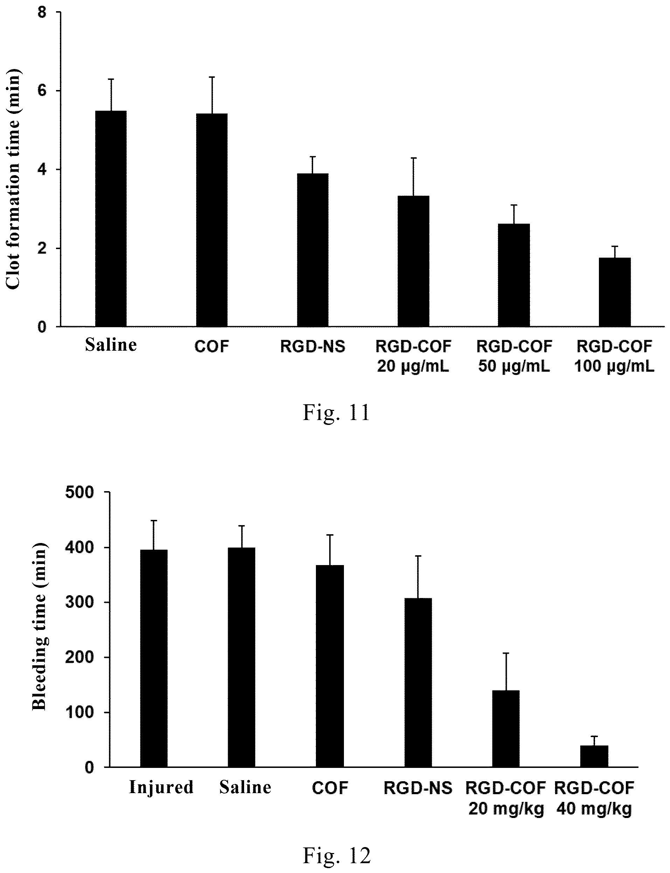

The results of blood clot formation time *in vitro* are shown in Fig. 11. It showed that RGD-COF significantly shorten the coagulation time of rat whole blood *in vitro* in a dose-dependent manner. The average clot formation time of whole blood of healthy rats *in vitro* was 5.5 min, and the mean of the clot formation time of COF blank carrier group prepared in Example 1 was 5.4 min, which indicated that COF itself had no effect on coagulation process. The cuboidal RGD-COF prepared in Example 1 reduced clot formation time by 39%, 52% and 68% in low (20 µg/ml), medium (50 µg/ml) and high dose groups (100 µg/ml), respectively, while the spherical RGD-NS prepared in Example 9 only reduced clot formation time by 29%, thus its hemostatic effect *in vitro* was far inferior to the cuboidal RGD-COF.

The low, medium and high dose groups of RGD-COF prepared in Example 2 reduced clot formation time by 32%, 49% and 61%, respectively.

The low, medium and high dose groups of RGD-COF prepared in Example 3 reduced clot formation time by 36%, 55% and 69%, respectively.

The low, medium and high dose groups of RGD-COF prepared in Example 6 reduced clot formation time by 37%, 48% and 65%, respectively.

### Example 11

Evaluation of hemostatic effect of RGD-COF *in vivo.* Nanoscale RGD-COF hemostatic drug for intravenous injection could only be injected intravenously after being dispersed with normal saline. The preparation method of nanoscale RGD-COF hemostatic drug for intravenous injection comprised the following steps:
(1) The cuboidal RGD-COF nanoparticles were prepared according to the methods of Example 1, Example 2, Example 3 and Example 4; and the spherical RGD-NS nanoparticles were prepared according to Example 9.
(2) Dispersion of nanoparticles: The nanoparticles were suspended in normal saline (1-10 mg/ml, preferably 2 mg/ml) to obtain nanoparticle dispersion.

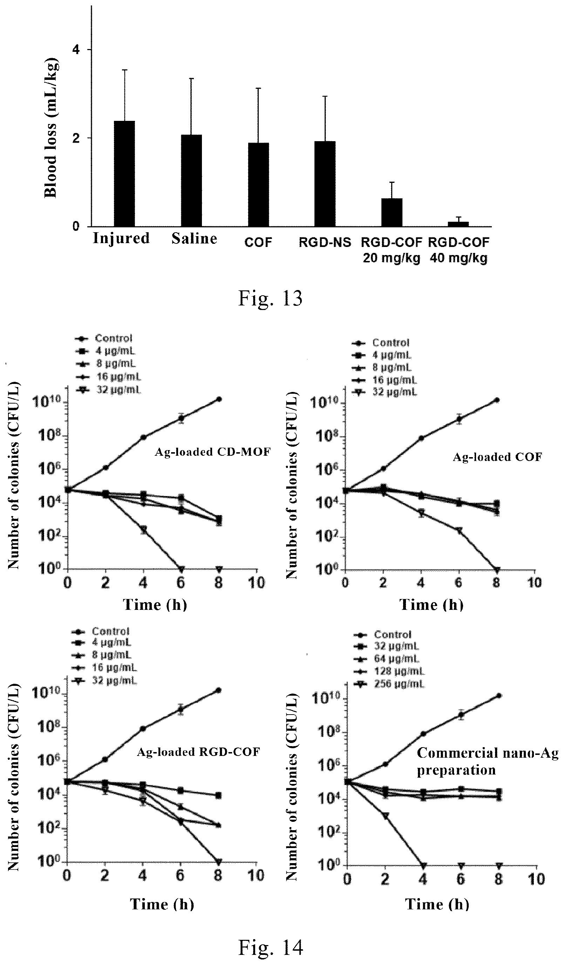

The hemostatic capacity of RGD-COF nanoparticles *in vivo* was evaluated by mouse tail transection model. Fifty healthy Kunming mice were randomly divided into 5 groups with 10 mice in each group. A blank control group (only tail transection, corresponding to "injured" in Figs. 12 and 13), saline group, COF group, RGD-NS group, RGD-COF low dose group (20 mg/kg, i.e. 20 mg/kg in Figs. 12 and 13) and RGD-COF high dose group (40 mg/kg, i.e. 40 mg/kg in Figs. 12 and 13) were set up respectively. Administration was via tail vein injection, single dose, and the dosage was 20-40 mg/kg body weight. The dosage volume was 10 mL/kg. 5 min after the administration, the tail was amputated 0.5 cm from the end of tail by a sharp scissor. The time was recorded immediately once the blood flowed out. The amputated tail was touched lightly with absorbent paper every 20 s, until the bleeding from the amputated tail stopped (without blood streak), this period was recorded as the bleeding time. The area of blood stain on the absorbent paper was calculated by Image Pro Plus software, and then the blood loss of mice was assessed.

The results of hemostatic experiment *in vivo* showed that RGD-COF significantly shortened the bleeding time and reduced the amount of bleeding in mice, and had a good hemostatic effect *in vivo.* As shown in Figs. 12 and 13, compared with the normal saline group or the COF carrier group, the low dose group (20 mg/kg) of RGD-COF prepared in Example 1 could shorten the bleeding time from 400s to 150s, which was shortened by 62.5%; and the blood loss was reduced from 2 mL/kg to 0.6 mL/kg, which was reduced by 70%. The high dose group (40 mg/kg) of RGD-COF prepared in Example 1 could shorten the bleeding time from 400s to 40s, which was shortened by 90%; and the blood loss was reduced to 0.2 mL/kg, which was reduced by 90%.

The low dose group of RGD-COF prepared in Example 2 could shorten the bleeding time from 400s to 155s, which was shortened by 61%; and the blood loss was reduced from 2 mL/kg to 0.8 mL/kg, which was reduced by 60%. The high dose group of RGD-COF prepared in Example 2 could shorten the bleeding time from 400s to 50s, which was shortened by 87.5%; and the blood loss was reduced to 0.12 mL/kg, which was reduced by 94%. However, the spherical RGD-NS prepared in Example 9 reduced bleeding time by only 23% and could not reduce blood loss. Therefore, the cuboidal RGD-COF had far more efficient hemostatic effect *in vivo* than that of spherical RGD-NS.

The high dose group of RGD-COF prepared in Example 3 could shorten the bleeding time from 400s to 52s, which was shortened by 7%; and the blood loss was reduced to 0.15 mL/kg, which was reduced by 92.5%.

The high dose group of RGD-COF prepared in Example 4 could shorten the bleeding time from 400s to 47s, which was shortened by 88.2%; and the blood loss was reduced to 0.17 mL/kg, which was reduced by 91.5%.

### Example 12

Micron-scale CD-MOF was prepared as in Example 6.

Silver loaded CD-MOF. 169 mg of silver nitrate was weighed and dissolved with acetonitrile to 100 ml in a volumetric flask to prepare 10 mmol/L silver nitrate solution. The micro-scale CD-MOF 600 mg was weighed and added into an EP tube, and 1.5 ml acetonitrile was added, mixed and ultrasonically treated for 10 min. Then 5 ml 10mmol/L silver nitrate solution was added, placed in the dark for 72 h. After washing with acetonitrile (10 ml) for 3 times and centrifuging at 4000 rpm for 5 min, and the precipitate was dried at 40°C for 12 h in a vacuum drying oven.

Micron-scale silver-loaded COF. 1g of micron-scale silver-loaded CD-MOF powder dried in vacuum at 80°C was weighed and added into a round bottom flask which was fixed on a magnetic stirrer. 12.85 mL N,N-dimethylformamide was added, and the mixture was heated at 80°C and stirred at 500 rpm. When the temperature of the reaction reached 60°C, 0.99 g of crosslinking agent diphenyl carbonate (the molar ratio of CD-MOF to crosslinking agent is 1: 6) and 0.5 ml of catalyst triethylamine were added. The mixture was reacted for 24 h, and centrifuged at 4000 rpm for 5 min. The precipitate was washed each twice with ethanol (10 ml), water (10 ml) and acetone (10 ml), respectively, and dried at 60°C in vacuum oven for 6 h. The obtained micron-scale silver-loaded COF had regular cubic shape with a particle size of 1-5 µm.

Silver-loaded RGD-COF: 1 g micron-scale silver-loaded COF and 21.7 mg 4-dimethylaminopyridine were weighed and added into a round bottom flask, 21.7 ml N, N-dimethylformamide was added and stirred evenly, and then 21.7 mg linear GRGDS, 13.36 mg carbamide and 86.95 uL triethylamine were added. The mixture was stirred on a magnetic stirrer (500 rpm) in dark at 37°C for 24 h, and centrifuged at 4000 rpm for 5 min. The precipitate was washed with N, N-dimethylformamide (10 mL), ethanol (10 mL), water (10 mL) and acetone (10 mL) once, respectively, and the obtained crystal was dried in vacuum at 60°C for 6 h. The obtained silver-loaded RGD-COF had regular cubic shape with a particle size of 1-5 µm, and the mass ratio of cyclodextrin framework material to RGD measured by HPLC is 1: 0.015.

Antibacterial effect: The MIC values of silver-loaded CD-MOF, silver-loaded COF and silver-loaded RGD-COF against *Escherichia coli* CMCC (B) 44102 were all 16 µg/ml, which had a better antibacterial effect than that of commercial preparation (Bangerjie^{®}) (128 µg/ml). It could be seen from the time-bactericidal curve (Fig. 14) that when the concentration of Ag was 4 µg/ml-16 µg/ml, the bactericidal effect of nano-silver on *Escherichia coli* CMCC (B) 44102 was weak, which was obviously enhanced when the concentration of Ag was greater than 16 µg/ml or increasing gradually. The nano-silver of 1.0 MBC (32 µg/ml) could well inhibit the growth of bacteria within 6-8 h. The MIC values of silver-loaded CD-MOF, silver-loaded COF and silver-loaded RGD-COF against *Staphylococcus aureus* CMCC (B) 26112 were all 128 µg/ml, and the antibacterial effects were similar to that of commercial preparation (128 µg/ml).

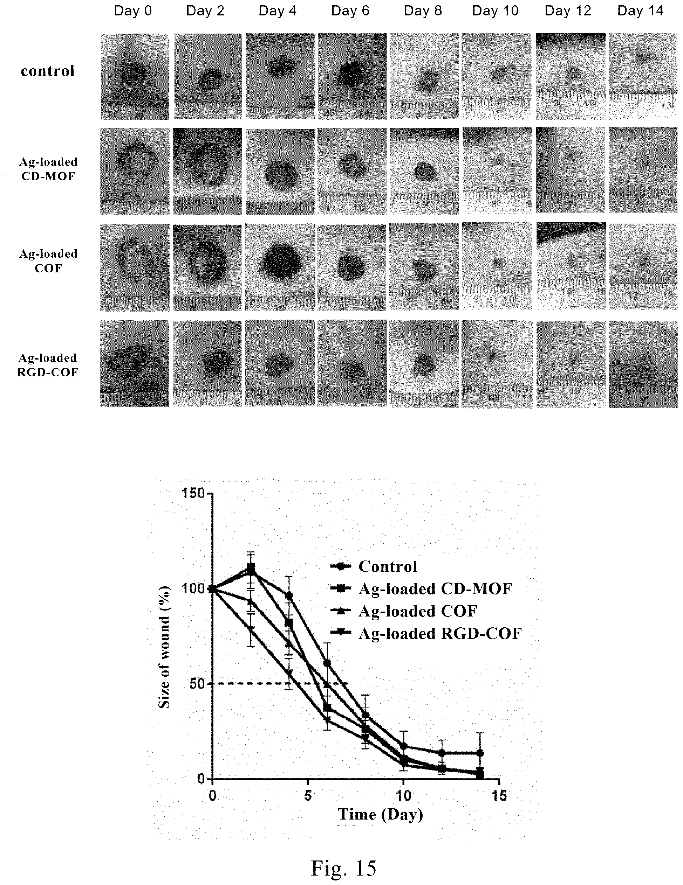

Wound healing effect (Fig. 15): Compared with the commercial silver nanoparticle reagent (control group), silver-loaded RGD-COF significantly accelerated the healing speed of epidermal wound in rats. As compared with silver-loaded CD-MOF and silver-loaded COF groups at the same time, silver-loaded RGD-COF had better healing effect on epidermal wounds in rats.

### Example 13

RGD-COF loaded with antithrombotic drugs: Seven antithrombotic drugs of aspirin, ticlopidine hydrochloride, cilostazol, clopidogrel bisulfate (type II), rivaroxaban, ozagrel hydrochloride, tirofiban hydrochloride were weighed and dissolved by adding 30 mL dry ethanol and ultrasonically treated for 10 min, respectively. Then 500 mg RGD-COF prepared in Example 1 was added (the molar ratio of the drug to RGD-COF was 2: 1), and stirred at 300 rpm at room temperature for 24 h to incubate and load the drug. After completion of drug loading, the mixture was centrifuged at 4000 rpm for 5 min, and the drug-loaded RGD-COF in lower layer was obtained. The drug loading was determined by HPLC as shown in Table 1.

**Table 1 Drug loading efficiency of nanoscale RGD-COF loaded with antithrombotic drugs**

| Drug name | Drug Loading of RGD-COF mg/mg (%) |
|---|---|
| Aspirin | 2.9 |
| Ticlopidine hydrochloride | 3.1 |
| Cilostazol | 1.1 |
| Clopidogrel bisulfate (type II) | 2.5 |
| Rivaroxaban | 2.3 |
| Ozagrel hydrochloride | 3.0 |
| Tirofiban hydrochloride | 3.2 |

### Example 14

RGD-COF loaded with hemostatic drugs: Three hemostatic drugs of tranexamic acid, aminocaproic acid and vitamin K1 were weighed and dissolved with 30 mL dry ethanol and ultrasonically treated for 10 min, respectively. Then 500 mg RGD-COF prepared in Example 1 was added (the molar ratio of drug to RGD-COF was 2: 1), and stirred at 400 rpm at 37°C for 12 h to incubate and load the drug. After completion of drug loading, the mixture was centrifuged at 4000 rpm for 5 min, the drug-loaded RGD-COF in lower layer was obtained. The drug loading determined by HPLC was 13.5%, 4.3%, and 2.1%, respectively.

### Example 15

RGD-COF loaded with anti-infective drugs: Four anti-infective drugs of sulfadiazine, ceftriaxone, amoxicillin and levofloxacin were weighed and dissolved with 30 mL dry ethanol and ultrasonically treated for 10 min, respectively. Then 500 mg of RGD-COF prepared in Example 1 was added (the molar ratio of RGD-COF to drug was 1: 1), and stirred at 200 rpm at room temperature for 48 h. After completion of drug loading, the mixture was centrifuged at 4000 rpm for 5 min, the drug-loaded RGD-COF in lower layer was obtained. The drug loading determined by HPLC was 3.8%, 3.3%, 4.6%, and 6.7%, respectively.

### Example 16

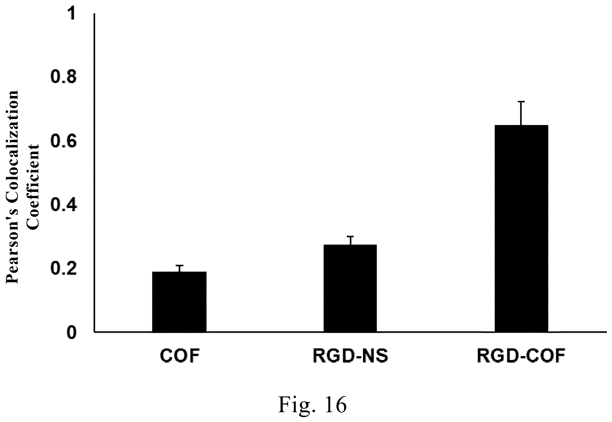

RGD-COF targeting thrombus *in vivo*: Formation of mouse mesenteric thrombus was induced by FeCl3, and activated platelets at thrombus site were labeled with rhodamine B injected in advance. After thrombosis, red Cy5 fluorescence labeled RGD-COF (40 mg/kg) prepared in Example 3 was injected into tail vein. It was observed under fluorescence microscope that red Cy5 fluorescence labeled RGD-COF could target and enrich in mesenteric thrombus. The co-localization coefficient of RGD-COF nanoparticles and activated platelets at thrombus was as high as 0.65, which was much higher than that of unmodified COF and spherical RGD-NS group (Fig. 16), indicating that cuboidal RGD-COF could highly target thrombus *in vivo.*

### Example 17

RGD-COF targeting bleeding site *in vivo*: The mouse tail transection model was utilized to evaluate the targeting efficiency of RGD-COF prepared in Example 2 to bleeding site *in vivo.* CY5-labeled COF, RGD-NS and RGD-COF nanoparticles (40 mg/kg) were injected into the tail vein of Kunming mice. 5 min later, the tail was amputated 0.5 cm from the end of tail by a sharp scissor to construct the mouse tail transection model. After 10 minutes of tail amputation, when the bleeding at the transected tail had stopped, the mouse tail was amputated 1 cm away from the first incision, and the mouse tail sample with a length of 1 cm was obtained. The fluorescence signal of RGD-COF aggregated at the transected tail was measured by small animal vivo imager. Because the RGD-COF could target activated platelets aggregated at the bleeding site, the fluorescence signal at the transected tail of RGD-COF group was 4 times of that in COF group, and 3 times of that in RGD-NS group, which indicated that the cuboidal RGD-COF could highly target and aggregate at the transected tail bleeding site, and had a better targeting efficiency *in vivo* than that of spherical RGD-NS.

All documents referred to in the present invention are incorporated by reference herein as if each document is individually incorporated by reference. Further, it should be understood that upon reading the above teaching of the present invention, various modifications or modifications may be made to the present invention by those skilled in the art, and those equivalents also fall within the scope defined by the appended claims of the present application.

## Claims

1. A cyclodextrin framework-RGD composition, wherein a mass ratio of cyclodextrin framework to RGD in the composition is 1: 0.001-1: 1, and preferably 1: 0.005-1: 0.5;
the cyclodextrin framework-RGD composition had a particle size of 10 nm-50 µm, preferably 50 nm-50 µm, and more preferably 100-500 nm or 1-5 µm.

2. The composition of Claim 1, which is a cuboidal cyclodextrin framework-RGD composition.

3. A preparation method of a cuboidal cyclodextrin framework-RGD composition, which comprises the steps of:
(1) providing a cuboidal cyclodextrin-organometallic framework (CD-MOF);
(2) crosslinking step: crosslinking the cuboidal cyclodextrin-metal organic framework of step (1) with a crosslinking agent to obtain a cyclodextrin framework (COF);
(3) RGD modification step: modifying RGD onto the cyclodextrin framework of step (2), thereby obtaining a cuboidal cyclodextrin framework-RGD composition (RGD-COF).

4. The preparation method of Claim 3, wherein the cross-linking step comprises the following sub-steps:
(2a) a dispersing step: dispersing the cuboidal cyclodextrin-metal organic framework in a organic solvent B to obtain a dispersing liquid 2a;
(2b) a step of adding crosslinking agent and the catalyst: under a crosslinking reaction temperature T, adding a crosslinking agent and a catalyst A into the dispersion liquid 2A, and obtaining the dispersion liquid 2b after a reaction time t1;
(2c) optionally, a cooling step: cooling the dispersion liquid 2b to obtain a cooled dispersion liquid 2b;
(2d) optionally, a step of terminating the reaction: adding a reaction terminator to the cooled dispersion 2b to obtain a dispersion 2d;
(2e) optionally, a centrifugal step: centrifuging the dispersion liquid 2d to obtain a crystal 2e;
(2f) optionally, a washing step: washing the crystal 2e to obtain a washed crystal 2f;
(2g) optionally, a drying step: drying the washed crystal 2f; and
(2h) obtaining a cuboidal cyclodextrin framework (COF).

5. The preparation method of Claim 3, wherein the RGD modification step comprises the following sub-steps:
(3a) a dispersing step: dispersing the cuboidal cyclodextrin framework (COF) and RGD in a organic solvent B to obtain a dispersing liquid 3a;
(3b) conjugating step: adding a catalyst B into the dispersion liquid 3a to conjugate the cuboidal cyclodextrin framework with RGD, wherein the reaction time is t2;
(3c) optionally, a centrifugation step;
(3d) optionally, a washing step;
(3f) optionally, a drying step;
(3g) obtaining the cuboidal cyclodextrin framework-RGD composition (RGD-COF).

6. The method of Claim 3, wherein the crosslinking agent is selected from the group consisting of peroxides, polyisocyanates, glycidyl ethers, diacids or polyacids, dialdehydes or polyaldehydes, compounds containing carbonyl groups, epoxides, acrylates, acyl chlorides, and combinations thereof.

7. The method of Claim 3, wherein,
the catalyst A is selected from the group consisting of 4-dimethylaminopyridine, triethylamine, 1-(3-dimethylaminopropyl)-3-ethylcarbodiimide or salts thereof, N,N'-succinimide carbonate, N-hydroxysuccinimide, pyridine, and combinations thereof; and is preferably triethylamine;
the organic solvent A is selected from the group consisting of dimethylformamide, tetrahydrofuran, methanol, acetonitrile, acetone, isopropanol, ethyl acetate, chloroform, n-hexane, ethanol and dichloromethane.

8. The method of Claim 3, wherein,
the catalyst B is selected from the group consisting of 4-dimethylaminopyridine, triethylamine, 1-(3-dimethylaminopropyl)-3-ethylcarbodiimide or salts thereof, N-hydroxysuccinimide, N,N'-succinimide carbonate, pyridine, and combinations thereof;
the organic solvent B is selected from the group consisting of dimethylformamide, tetrahydrofuran, methanol, acetonitrile, acetone, isopropanol, ethyl acetate, chloroform, n-hexane, ethanol, dichloromethane, and combinations thereof.

9. A drug-loaded cuboidal cyclodextrin framework-RGD composition, wherein the cuboidal cyclodextrin framework-RGD composition is the composition of claim 1 or 2, or is prepared by the method of claim 3, and the drug is selected from the group consisting of an antibacterial drug, a hemostatic drug, an antithrombotic drug, an anti-infective drug, and combinations thereof.

10. A use of an active ingredient, wherein the active ingredient is selected from the group consisting of:
(i) a cyclodextrin framework-RGD composition of claim 1 or 2;
(ii) a drug-loaded cuboidal cyclodextrin framework-RGD composition of claim 9;
(iii) a cyclodextrin framework (COF);
(iv) any combination of (i), (ii) or (iii);
wherein the active ingredient is used for:
(a) preparation of a drug-loading material;
(b) preparation of a therapeutic and/or diagnostic reagent or kit;
(c) preparation of a hemostatic drug and/or material;
(d) preparation of an anti-infective drug and/or material;
(e) preparation of an antimicrobial agent and/or material;
(f) preparation of a medicament and/or material for promoting wound healing; or
(g) preparation of a medicament and/or material for the prevention and/or treatment of thrombosis.

11. A pharmaceutical composition comprising:
(1) an active ingredient which is the cyclodextrin framework-RGD composition of claim 1 or 2 or the drug-loaded cuboidal cyclodextrin framework-RGD composition of claim 9; and
(2) a pharmaceutically acceptable carrier.
